# EUROPÄISCHE PATENTANMELDUNG

(11) **EP 4 613 476 A1**
(43) Veröffentlichungstag der Anmeldung: **10.09.2025**
(21) Anmeldenummer: 25161598.5
(22) Anmeldetag: 04.03.2025
(51) Int. Cl.: B32B 9/04, B32B 3/06, B32B 5/02, B32B 5/06, B32B 7/02, B32B 7/05, B32B 9/02

(54) **MEHRLAGENVERBUNDELEMENT**

(30) Priorität: 04.03.2024 LU 506504
(71) Anmelder: Wirtz Meyer GmbH, 02763 Zittau (DE)
(72) Erfinder: MEYER, Oscar, 02763 Zittau (DE); WIRTZ, Friedrich, 47199 Duisburg (DE)
(74) Vertreter: Gruner, Leopold Joachim

(57) **Zusammenfassung**

Die vorliegende Erfindung offenbart ein Mehrlagenverbundelement zur Verwendung als Leichtbauelement für konstruktive und isolierende Anwendungen, aufweisend ein Mehrlagengewebe, umfassend zumindest zwei mit Abstandselementen verbundene Gewebelagen, sowie einen Myzelverbund, der mit den Gewebelagen in Teilbereichen oder flächendeckend form- und/oder stoffschlüssig verbunden ist, wobei der Myzelverbund im Zwischenraum, der durch die der Gewebelagen aufgespannt wird, wirkverbunden angeordnet ist. Weiterhin betrifft die Erfindung ein Verfahren zur Herstellung des Mehrlagenverbundelements und ein Mehrlagenverbundsystem, welches das Mehrlagenverbundelement umfasst.

## Beschreibung

### TECHNISCHES GEBIET

Die vorliegende Erfindung betrifft das technische Gebiet der Verbundwerkstofftechnik, konkret ein Mehrlagenverbundelement zur Verwendung als Leichtbauelement für tragende, konstruktive und isolierende Anwendungen, aufweisend ein Mehrlagengewebe, umfassend zumindest zwei mit Abstandselementen verbundenen Gewebelagen, sowie ein Myzelverbund, der mit den Gewebelagen in Teilbereichen oder flächendeckend form- und/oder stoffschlüssig verbunden ist, wobei der Myzelverbund im Zwischenraum, der durch die aufgespannten Gewebelagen gebildet wird, wirkverbunden angeordnet ist. Weiterhin betrifft die Erfindung ein Verfahren zur Herstellung des Mehrlagenverbundelements und Mehrlagenverbundsystem, welches das Mehrlagenverbundelement umfasst.

### STAND DER TECHNIK

In den vergangenen zehn Jahren wurde die Bauindustrie stark unter Druck gesetzt, da die herkömmlichen Baumaterialien wie Zement, Ziegel, sowie Verkleidungs- und Trennwandmaterialien mit den stetig wachsenden Anforderungen an den Umwelt- und Klimaschutz nicht Schritt halten konnten. Die Herstellung dieser traditionellen Baumaterialien erfordert eine erhebliche Menge an Energie, führt zur Erschöpfung natürlicher Ressourcen und verursacht Luft-, Boden- und Wasserverschmutzung. Bis zu 36 % des gesamten Energieverbrauchs über die Lebensdauer eines typischen Wohnhauses entfallen auf die Gewinnung oder Ernte von Rohstoffen, ihre Verarbeitung, den Transport und den Bau des Gebäudes. Obwohl Niedrigenergiehäuser während ihrer Nutzung weniger Energie verbrauchen, ist ihr Bau weniger umweltfreundlich (bis zu 46 % des gesamten Energieverbrauchs eines Wohngebäudes entfallen auf die Bauphase), da für die Herstellung von zusätzlicher Isolierung, dichteren Materialien und verwendeten Technologien mehr Energie benötigt wird.

Zu den klassischen Leichtbau- und Dämmstoffen zählen vor allem chemische Schäume, vor allem auf Polyurethanbasis, sowie Polystyrol, Mineral- und Glaswolle oder Leichtbaustoffe wie Bimsstein, Blähperlit und Schaum- oder Leichtbeton. Diese Materialien bieten zwar eine hohe Stabilität und Wärmedämmleistung, jedoch sind sie mit verschiedenen Nachteilen behaftet. Ein großer Nachteil dieser Materialien ist ihre langfristige Umweltauswirkung, insbesondere ihre schlechte biologische Abbaubarkeit und der Beitrag zur globalen Erwärmung durch Freisetzung von Treibhausgasen bei Herstellung und Abbau. Zudem sind die Herstellungsprozesse dieser Schaumstoffe energieintensiv. Die Abhängigkeit von fossilen Brennstoffen für die Produktion dieser Materialien verstärkt nicht nur die Erschöpfung der natürlichen Ressourcen, sondern trägt auch zur Verschärfung der Umweltproblematik bei Angesichts der dringenden Notwendigkeit, nachhaltige und umweltfreundliche Bauweisen zu fördern, wird deutlich, dass alternative Materialien und Technologien erforderlich sind, um die Bauindustrie zu revolutionieren und einen Beitrag zur Reduzierung des ökologischen Fußabdrucks zu leisten.

Aus diesem Bereich sind verschiedene konstruktive Anordnungen bekannt, um Leichtbauelemente in Verbundtechnik bereitzustellen. Beispielsweise beschreibt die europäische Patentschrift EP 1305159 B1 ein Leichtbauelement, das aus einem textilen Steggewebe und mindestens einem partikelförmigen, gequollenen thermoplastischen Polymermaterial besteht. Dieses Material wird derart zwischen den Stegen des Gewebes platziert, dass es die textilen Verbindungsfasern spannt, wobei die Partikel des gequollenen Materials zumindest teilweise untereinander vernetzt sind. Das resultierende Leichtbauelement zeichnet sich durch eine herausragende Stabilität bei gleichzeitig minimalem Gewicht aus. Ein wesentlicher Nachteil dieser Konfiguration liegt jedoch im CO₂-Fußabdruck der genutzten Polymermaterialien sowie in der Abhängigkeit von fossilen Rohstoffen. Ein weiterer Nachteil ist die komplexe Entsorgungsproblematik im Zusammenhang mit der langfristigen Persistenz in der Umwelt.

Die rasch wachsende Weltbevölkerung hat auch zu einer steigenden Nachfrage nach Nahrungsmitteln und einer erhöhten landwirtschaftlichen Produktion geführt, was wiederum zur Erzeugung von landwirtschaftlichen Nebenprodukten und Abfällen wie Zuckerrohrbagasse, Reishülsen, Baumwollstängeln, Stroh und Halmen beiträgt. Allein in Indien und Südostasien fallen jedes Jahr bis zu 1 Milliarde Tonnen Biomasse an. Diese minderwertigen landwirtschaftlichen Nebenprodukte und Abfälle haben nur begrenzte Verwendungsmöglichkeiten, da sie hauptsächlich als Düngemittel, Tiereinstreu und Füllstoffe für Baumaterialien und den Straßenbau verwendet werden, aber größtenteils als Abfall entsorgt oder verbrannt werden, wodurch Kohlendioxid und andere Treibhausgase freigesetzt werden.

In den letzten zehn Jahren hat das vegetative Wachstum fadenförmiger Pilze, auch Myzel genannt, als neue Form der Niedrigenergie-Biofabrikation und des Upcycling von Abfällen zunehmendes akademisches und kommerzielles Interesse auf sich gezogen. Das Myzel bindet organische Materialien durch ein Netz von Hyphen-Mikrofilamenten in einem natürlichen biologischen Prozess, der zur Herstellung von geringwertigen Materialien wie Verpackungen und höherwertigen Verbundstoffen aus problematischen landwirtschaftlichen und industriellen Abfällen mit geringem oder ohne Handelswert genutzt werden kann.

Dennoch gibt es mehrere Einschränkungen bei der aktuellen Anwendung und Verwendung von Myzelmaterialien, die hauptsächlich auf ihren typischerweise schaumartigen mechanischen Eigenschaften, hoher Wasseraufnahme und vielen Lücken in der Dokumentation der Materialeigenschaften beruhen. Weiterhin mangelt es bisher an Verbundmaterialien, die die attraktiven umweltfreundlichen Eigenschaften der Myzelmaterialien mit notwendigen mechanischen Eigenschaften verbinden, die für die Anwendung im technischen Gebiet des Leichtbaus und der Bauindustrie notwendig sind.

Im Stand der Technik sind einige Verfahren zur Herstellung von Verbundmaterialien auf Basis von Myzel beschrieben.

Ein Verfahren zur Kultivierung von Myzel auf einem Lignozellulosesubstrat, welches mit Nährstoffen angereichert ist, wird im Patent US 9914906 B2 offenbart. Das daraus resultierende Material wird in einzelne Partikel zerteilt, diese werden im Anschluss in unterschiedliche Materialien, beispielsweise in Bioharzen eingesetzt. Diese Patentschrift offenbart also nur die Verwendung von Myzelkompositen als Füllstoffpartikel für Verbundwerkstoffe.

Die europäische Patentschrift EP 3709791 A1 beschäftigt sich mit der Beeinflussung der Wachstumsrichtung des Myzels. Dabei wird das Myzel in einer speziellen Kammer kultiviert, in die ein gerichteter Luftstrom eingeleitet wird. Durch Variation der Luftfeuchtigkeit und des Luftstroms während der Kultivierung kann die Wachstumsrichtung des Myzels gesteuert werden, um eine gleichmäßige Verteilung des Myzels im Material zu erreichen. Ziel dieses Verfahrens ist es, Materialien zu entwickeln, die Leder, Textilien oder Schaumstoffe ersetzen können. Dabei wird jedoch keine Lösung aufgezeigt, wie die Stabilitätsprobleme von reinen Myzelverbünden überwunden werden könnten.

Die US-Patentschrift US 10125347 B2 beschreibt ebenfalls ein Verfahren, um die Expression bestimmter Hyphenstrukturen in Pilzen zu stimulieren. Dies erfolgt durch die Exposition gegenüber anderen Mikroorganismen, die die fadenförmigen Pilze dazu anregen, spezifische Hyphenmorphologien zu entwickeln. Das resultierende Material weist ein Myzel mit reduzierter Dichte auf.

Die europäische Patentschrift EP 2702137 B1 beschreibt eine Methode zur Herstellung von getrockneten Myzelelementen, die bei Bedarf miteinander verbunden werden können. Zunächst wird ein Myzelelement gemäß bekannten Verfahren hergestellt und anschließend getrocknet. Wenn eine Verbindung der Elemente gewünscht ist, werden mindestens eine Seite von mindestens zwei Myzelelementen angefeuchtet und mit Nährstofflösung versetzt. Dann werden die angefeuchteten Seiten der Myzelelemente zusammengebracht und das Pilzwachstum verbindet die beiden Elemente. Nach einer erneuten Trocknung, bei der der Pilz inaktiviert wird, können die erhaltenen Elemente weiterverarbeitet werden. Dieses Verfahren eignet sich besonders, wenn eine große Materialfläche benötigt wird und die kleineren Einheiten transportiert und erst am Zielort zusammengesetzt werden sollen. Eine spezielle Festigkeit des resultierenden Myzels wird nicht beschrieben.

Die deutsche Patentschrift DE 10 2021 134 036 A1 befasst sich damit, ein myzelbasiertes Material bereitzustellen, welches besonders stabil ist und eine erhöhte Festigkeit im Vergleich zu bekannten myzelbasierten Materialien aufweist. Der Inhalt der Offenlegung bezieht sich jedoch auf den Myzel-Verbundstoff an sich, ohne weitere Aussagen zu möglichen Verbundwerkstoffen zu machen.

Die internationale Patentschrift WO 2019/226823 A1 beschreibt ein Verfahren mit dem ein Myzelmaterial hergestellt werden kann, das Materialstärken von über 15 cm und speziell eine Materialstärke von 60 bis 71 cm aufweist. Dies wird erreicht, indem das wachsende Myzel-Biomaterial mit frischem Sauerstoff versorgt wird, während Abwärme und Kohlendioxid durch Zwangsbelüftung abgeführt werden. In einer ersten Phase der Pilzexpansion wird befeuchtete Luft mit einer programmierten Temperatur nach oben und durch ein mit Pilzen geimpftes Substrat aus diskreten Partikeln geleitet, damit sich das Pilzinokulum ausdehnen und das Substrat beherrschen kann. Dem geimpften Gemisch werden Nährstoffe zugesetzt und eine zweite Phase der Pilzexpansion durchgeführt, in der befeuchtete Luft bei einer programmierten Temperatur nach oben und durch das mit Nährstoffen angereicherte geimpfte Pilzsubstrat geleitet wird, damit das Pilzinokulum die einzelnen Partikel zu einem selbsttragenden Biokomposit verbinden kann. Dabei wird jedoch kein Verbundwerkstoff offenbart.

Die internationale Patentschrift WO 2012/071589 A2 offenbart ein Verfahren zur Züchtung von organischen Baustoffen in Form eines formbaren Substrats, das für eine breite Palette von Fertigungs- und Bauanwendungen eingesetzt werden kann. Insbesondere berücksichtigen die Ausführungsformen eine Vielzahl von Pilzformkörpern, die vorzugsweise aus einem Pilzinokulum gezüchtet und mindestens einmal während des Wachstumsprozesses mechanisch komprimiert werden, sowie die Integration von Strukturträgern in die Pilzstruktur. Die Erfindung stellt ein Pilzsubstrat zur Verfügung, das geformt und einfach und kostengünstig zu präzisen geometrischen Spezifikationen vorbearbeitet werden kann.

Die internationale Patentschrift WO 2018/014004 A1 stellt ein Verfahren zur Bildung von Pilzmaterialien und Pilzgegenständen vor, in dem Pilzgewebe auf einem Nährstoffträger gezüchtet wird, um es anschließend gezielt durch ein poröses Material wachsen zu lassen, so dass ein Teil des Pilzgewebes ein erstes Pilzmaterial mit ersten Pilzhyphen umfasst. Daraufhin wird eine Änderung der Zusammensetzung oder des Wachstumsmusters mindestens einiger der ersten Pilzhyphen veranlasst, ein Teil des ersten Pilzmaterials wird von dem Nährstoffträger abgetrennt und man erhält ein zweites Pilzmaterial mit zweiten Pilzhyphen. Aus dem ersten und dem zweiten Pilzgegenstand wird durch Fördern des Schmelzwachstums zwischen dem ersten und dem zweiten Pilzgegenstand ein fertiges Pilzmaterial gewonnen.

Die internationale Patentschrift WO 2020/082044 offenbart ein Verfahren mit einem Myzelium-Wachstumsbett für die Aufzucht von festem, substratgebundenem Myzel, durch das der Myzelverbund leicht und einfach entfernt werden kann. Dies wird durch die Verwendung einer Perforationsschicht erreicht, die zwischen dem Myzelium-Substrat und dem Myzelium-Verbundstoff eingebettet ist, um eine gleichmäßige strukturelle Schwäche zu erzeugen und dadurch die Erntefähigkeit des Myzels aus dem Substrat durch eine stark reduzierte und gleichmäßige Reißfestigkeit zu verbessern. Die Perforationsschicht, durch die das Myzel wächst, ermöglicht die kontrollierte Extrusion einer Matrix aus kolonialen Zellen, die sich leicht und gleichmäßig von dem darunter liegenden Myzelsubstrat ablösen lassen.

Darüber hinaus offenbaren Patentschriften Dämmstoffe mit strukturellen Gerüsten oder in speziellen Formen, wie etwa in der internationalen Patentschrift WO 2017/132523 A1, die sich auf biologisch abbaubare Dämmstoffe bezieht, die ein strukturelles Gerüst und mindestens einen temperaturbeständigen Pilz umfassen. Zusätzlich wird auch ein Verfahren zur Herstellung und Verwendung von biologisch abbaubaren Isoliermaterialien, die ein strukturelles Gerüst und mindestens einen temperaturresistenten Pilz umfassen vorgestellt. Das europäische Patentschrift EP 2094856 B1 stellt ein Verfahren zur Herstellung von organischen Isolierungen, organischen Verpackungen, organischen Kühlern, organischen Pflanzentöpfen und dergleichen vor.

Die Patentschrift US 2023/294369 A1 offenbart ein Baumodul in Form eines Sandwich-Panels, das Teil einer Dachstruktur eines Hauses ist. Hierbei wird ein Verfahren zur Herstellung eines solchen Sandwich-Panels offenbart, in dem ein inokuliertes Myzel in den Zwischenraum eingebracht und nach einer Wachstumsphase getrocknet wird.

Die Patentschrift DE 10 2020 134625 A1 beschreibt ein flächiges Dämm- und/oder Konstruktionselement aus einem biologisch erzeugten Kompositmaterial, dass insbesondere zur Wärme- und/oder Schalldämmung dient. Hierbei umfasst das Element ein partikuläres Substrat (2) und ein das Substrat zumindest im Wesentlichen umgebendes und/oder durchdringendes Myzel (3) eines Pilzes. Weiterhin wird die Möglichkeit einer Armierung durch zusätzlich angeordnete Platten oder Gewebe offenbart. Nachteilig geht hierbei nicht hervor, ob uind wie eine Wirkverbindung zwischen Myzelmaterial und einer derartigen Armierung erfolgt.

Aus dem Stand der Technik geht kein Myzelverbundwerkstoff hervor, der über ausreichend hohe Festigkeit, gute Dämmeigenschaften, niedriges Gewicht und geringe Wasserabsorbation verfügt, um herkömmliche Verbundstoffe und Sandwichelemente, beispielsweise aus Polyurethan, zu ersetzen. Insbesondere gibt es im Stand der Technik keinen Ansatzpunkt, der geeignet wäre, dem Fachmann einen Weg zu strukturstabilen, Gewebe-Myzel-Verbundsystemen aufzuzeigen.

Der Stand der Technik weist dazu einige Probleme auf. Zum einen gibt es ein Delaminierungsproblem bei MyzelVerbundwerkstoffen. Zum anderen schrumpfen Myzelmaterialien stark in der Z-Richtung. Um höhere Festigkeiten bei Myzelmaterialien zu erreichen, werden diese außerdem verdichtet. Dies führt allerdings zu schlechteren Dämmeigenschaften und höheren Wasserabsorbationswert, was wiederum den breiten Einsatz von Myzelverbundwerkstoffen erschwert.

### AUFGABE

Aufgabe der vorliegenden Erfindung ist es, einen Verbundwerkstoff, insbesondere Platten und andere Arten von Körpern, bereitzustellen, die einerseits leicht, andererseits stabil und auch als selbsttragende Wände und Decken, Träger, Dächer und Laubengänge sowie Formkörper für den Einsatz in Kraftfahrzeugen, Schiffen und Flugzeugen geeignet sind, der die Vorteile des textilen Leichtbaus, mit mehrwandigen Geweben oder Geweben mit verklebten Deckschichten mit herkömmlichen Dämmstoffen wie geschäumten Polyurethanen, die eine hohe Umweltverträglichkeit aufweisen, und die Vorteile nachwachsender Rohstoffe verbindet. Gleichzeitig sollen die verwendeten Materialien recycelbar und umweltfreundlich sowie kostengünstig in der Herstellung sein, um den Einsatz des Verbundwerkstoffs insbesondere auch im zivilen Bau- und Transportbereich zu ermöglichen.

### LÖSUNG

Die Aufgabe wird durch ein Mehrlagenverbundelement gemäß Anspruch 1 gelöst. Weiterhin wird die Aufgabe durch ein Verfahren zur Herstellung des Mehrlagenverbundelements wie hierhin offenbart gelöst.

Weitere vorteilhafte Ausgestaltungen und Weiterbildungen ergeben sich aus den Unteransprüchen sowie aus der Beschreibung unter Bezugnahme auf die Figuren.

### ALLGEMEINE VORTEILE

Die Realisierung der Aufgabe mittels eines Mehrlagenverbundelements, welches ein Mehrlagengewebe beinhaltet, das wiederum aus mindestens zwei räumlich voneinander getrennten, durch Abstandselemente miteinander verbundenen Gewebelagen besteht, die einen Zwischenraum zwischen sich aufspannen, in dem ein Myzelverbund positioniert ist, ermöglicht eine Synthese der Vorteile konventioneller Verbunddämmstoffe, die auf Mehrlagengeweben mit synthetischen Dämmmaterialien wie Polyurethan basieren, mit den ökologischen Vorzügen. Zu diesen gehören die biologische Abbaubarkeit, CO₂-Effizienz, die Verringerung von Umwelttoxinen und das Potential für eine kreislaufwirtschaftliche Verwendung, welche charakteristisch für Myzel-basierte Werkstoffe sind. Diese innovative Kombination fördert nicht nur eine nachhaltigere Bauweise durch die Nutzung regenerativer Ressourcen, sondern trägt auch dazu bei, die Umweltbelastung zu reduzieren, indem sie eine ökoeffiziente Alternative zu herkömmlichen Dämmmaterialien bietet.

Eine bedeutende Errungenschaft der Erfinder besteht darin, erkannt zu haben, dass die Problematik der Delamination und Schrumpfung, welche in der Vergangenheit bei der Nutzung von myzelbasierten Materialien auftrat, effektiv vermieden werden kann. Dies wird erreicht, indem der Myzelverbund entweder teilweise oder vollständig form- und/oder stoffschlüssig mit mindestens zwei gegenüberliegenden Gewebelagen des Mehrlagengewebes sowie form- und/oder stoffschlüssig mit den Abstandselementen verbunden wird. Die Umsetzung dieser Erkenntnis, insbesondere durch das erfindungsgemäße Verfahren zur Herstellung des Mehrlagenverbundelements in Kombination mit speziell ausgewählten Mehrlagengeweben, die das Durchwachsen des Myzels während der Inkubationsphase ermöglichen, führt zur Ausbildung einer Wirkverbindung mit den Gewebelagen und Abstandselementen. Diese Verbindung unterdrückt technisch die Delamination und repräsentiert somit eine signifikante Verbesserung gegenüber bisherigen Ansätzen. Die Implementierung dieser Methodik erlaubt es, die strukturelle Integrität und die Langzeitstabilität myzelbasierter Verbundwerkstoffe zu erhöhen und stellt damit einen wesentlichen Fortschritt in der Materialwissenschaft dar.

Ein herausragender Vorteil der erfindungsgemäßen Anordnung liegt in der signifikant gesteigerten Stabilität des Mehrlagenverbundelements, insbesondere verglichen mit Myzelverbünden, die aus dem Stand der Technik bekannt sind. Diese Stabilität resultiert aus dem synergetischen Zusammenspiel zwischen dem Myzelverbund, der eine hohe Widerstandsfähigkeit gegen Druckbelastungen aufweist, und den durch Abstandselemente verstärkten Gewebelagen. Während des Inkubationsprozesses bieten die Gewebelagen effektiven Widerstand gegen die Expansion des Myzelverbunds und tragen im ausgereiften Verbundelement dazu bei, Zugbelastungen entlang der vertikalen Z-Achse aufzunehmen. Gleichzeitig sind die Gewebelagen dazu ausgerüstet, um Zugkräfte in den horizontalen X- und Y-Ebenen zu absorbieren. Diese multidirektionale Belastbarkeit gewährleistet eine umfassende strukturelle Integrität des Verbundelements, was es für eine Vielzahl von Anwendungen, insbesondere in konstruktiven Leichtbauelementen oder Dämmelementen mit struktureller Belastung prädestiniert.

### AUSFÜHRLICHE BESCHREIBUNG

### Mehrlagenverbundelement

Die Erfindung betrifft ein Mehrlagenverbundelement als Leichtbauelement für tragende und isolierende Anwendungen, bevorzugt zur Verwendung als Leichtbauelement für tragende und isolierende Anwendungen, umfassend zumindest
- ein Mehrlagengewebe, umfassend zumindest zwei zueinander beabstandete, mit Abstandselementen verbundene Gewebelagen, die dazu eingerichtet sind einen Zwischenraum zwischen den verbundenen Gewebelagen aufzuspannen,
- ein Myzelverbund, umfassend oder bestehend aus ein(em) Substrat und ein das Substrat durchdringendes Myzel,
wobei der Myzelverbund im Zwischenraum, der durch die zumindest zwei Gewebelagen aufgespannt wird, angeordnet ist, und wobei der Myzelverbund jeweils in Teilbereichen oder vollflächig form- und/oder stoffschlüssig mit zumindest zwei gegenüberliegenden Gewebelagen des Mehrlagengewebes und form- und/oder stoffschlüssig mit den Abstandselementen verbunden ist, wobei bevorzugt der Myzelverbund in Teilbereichen die äußere Schicht des Mehrlagenverbundes darstellt, wobei der Myzelverbund in diesen Teilbereichen die äußere Gewebelage durchdringt. Durch diese Anordnung kann die erfindungsgemäße Wirkverbundenheit zwischen dem Myzelverbund und dem Mehrlagengewebe geschaffen werden, der vorteilhaft eine Delamination oder sonstige Trennung verhindert. Es ist eine besondere Leistung der Erfinder, mit mehrlagigen Geweben im Zusammenspiel mit bekannten, aber mechanisch benachteiligten Myzelverbünden eine Materialkombination gefunden zu haben, die eine vorteilhafte, nachwachsende, CO₂-neutrale und isocyanatfreie Alternative zu bestehenden Leichtbaumaterialien darstellt.

Im Rahmen der vorliegenden Erfindung wird unter einem "Mehrlagenverbundelement", auch als "Verbundelement" bezeichnet, die konstruktive wirkverbundene Gesamtheit verstanden, die aus mehreren, durch spezifische Abstandselemente miteinander verbundenen Gewebelagen besteht, in Kombination mit einem Myzelverbund angeordnet, der zwischen ihnen angeordnet ist, um eine einheitliche Struktur zu bilden, wie hierhin beschrieben. Das Mehrlagenverbundelement kann in verschiedenen Formen, wie Ziegeln, Platten, Blöcken oder anderen geometrischen Formaten, ausgestaltet sein, abhängig von den spezifischen Anforderungen der jeweiligen Anwendung. Charakteristisch für das Mehrlagenverbundelement ist seine räumliche Ausdehnung, die jeweiligen Anwendungsbedürfnisse zugeschnitten sein kann. Diese Ausdehnungen sind so gestaltet, dass sie die funktionellen und strukturellen Anforderungen des Einsatzbereichs optimal erfüllen, sei es in Bezug auf Belastbarkeit, Isolation, Feuerresistenz oder andere spezifische Eigenschaften. Durch diese modulare und anpassungsfähige Konzeption ermöglicht das Mehrlagenverbundelement eine vielseitige Nutzung in verschiedenen Bereichen, von der Bauindustrie bis hin zu spezialisierten technischen Anwendungen, wobei es stets durch eine hohe Effizienz und Leistungsfähigkeit gekennzeichnet ist.

Besonders bevorzugt fungiert der Myzelverbund als erste äußere Schicht des Mehrlagenverbundelements, indem er durch zumindest Teile, insbesondere Teilbereiche der externen Gewebelagen, bevorzugt einer äußeren Gewebelage hindurchwächst. Diese Durchdringung bewirkt einen form- und/oder stoffschlüssig, besonders bevorzugt einen stoffschlüssigen Verbund zwischen den Gewebelagen und dem Myzelverbund. Die Feinabstimmung des Abstandes zwischen den gespannten Gewebelagen und einen Abdeckmittel (Abdecken der Grenzflächen) vor dem Inkubationsschritt des erfindungsgemäßen Verfahrens ermöglicht es, das Hervorstehen des Myzelverbundes über die äußeren Gewebelagen präzise zu kontrollieren und zu regulieren, bevorzugt ist dieser Abstand so gewählt, dass dieser zwischen 0 mm und 20 mm Abstand zur äußeren Gewebelage aufweist.

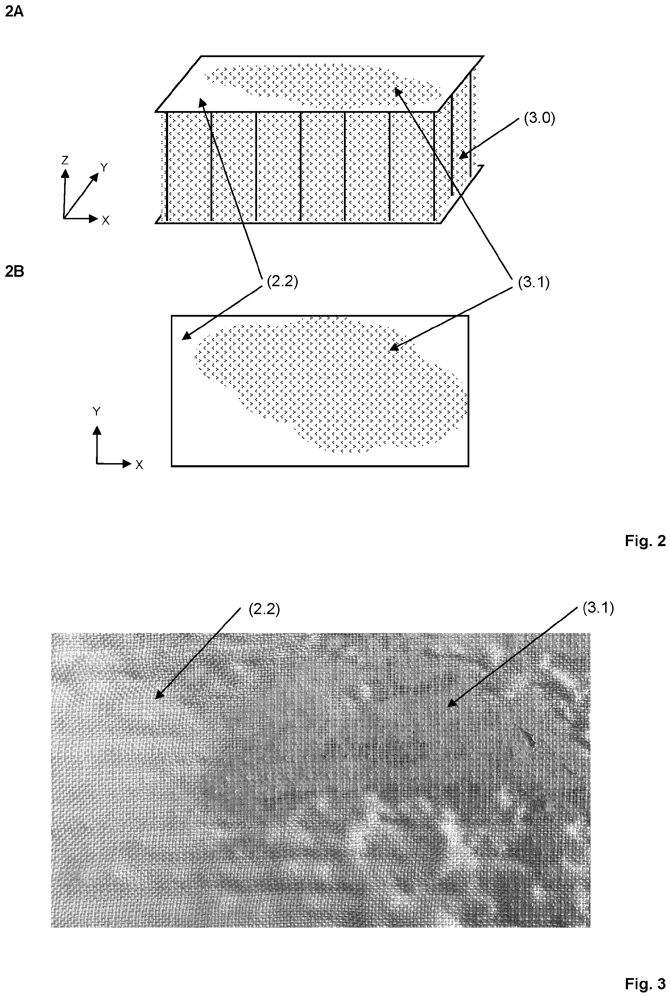

In einigen besonders bevorzugten Ausführungsformen, in denen der Abstand so gewählt ist, dass dieser zwischen 0mm und 20mm Abstand zur äußeren Gewebelage vor dem Inkubationsschritt des erfindungsgemäßen Verfahrens beträgt, wodurch das Hervorstehen des Myzelverbundes im Bereich von 0 mm und 20 mm beträgt. 0mm bedeutet hierbei, dass der Myzelverbund nicht über die äußere Gewebelage hervorsteht, wobei dieser dennoch zumindest formschlüssig durch Teile der Gewebelage dringt, jedoch nur in den technisch bedingten Toleranzbereich in dem durch die Webmuster entstandenen Vertiefungen des Gewebes herausragt. Beispielsweise ist ein solches Beispiel in Fig. 3 zu sehen, in der die Erhebungen des Webmusters der äußeren Gewebelage die Auflagefläche des Abdeckmittels darstellen und das Hervorstehen des Myzelverbundes auf 0 mm limitiert ist, doch dennoch die äußere Gewebelage durchdringt.

Besonders bevorzugt ist der Anteil, auch als Teilbereich bezeichnet, an durchwachsenen Gewebelage der äußeren Außenlage des Verbundelementes zwischen 10 und 100%, bevorzugter zwischen 20 und 100% der Gesamtoberfläche. Dies gewährleistet, dass die Teilbereiche form- und/oder stoffschlüssig mit zumindest zwei gegenüberliegenden Gewebelagen des Mehrlagengewebes verbunden ist und insbesondere die äußere Gewebelage vor Delaminierung geschützt ist. Durch diese Anordnung kann die erfindungsgemäße Wirkverbundenheit zwischen dem Myzelverbund und dem Mehrlagengewebe geschaffen werden, der vorteilhaft eine Delamination oder sonstige Trennung, hierbei insbesondere der äußeren Gewebelagen verhindert.

Im Allgemeinen bezieht sich "stoffschlüssig" eine Verbindungsart, bei der Materialien auf molekularer oder chemischer Ebene miteinander verbunden sind. Dies bedeutet insbesondere im Kontext dieser Erfindung, dass die Verbindung durch eine materielle Kohäsion zwischen den beteiligten Komponenten, wie dem Myzelverbund und den Gewebelagen oder dem Myzelverbund und den Abstandselementen, zustande kommt. Dies ist insbesondere in bevorzugten Ausführungsformen der Fall, in dem die Gewebelagen aus einer Naturfaser, insbesondere Baumwolle, Flachs, Hanf, Jute, Sisal, Kokosfaser oder Wolle bestehen. Stoffschlüssige Verbindungen entstehen durch chemische Reaktionen, Adhäsion oder Diffusion, wodurch eine dauerhafte und feste Verbindung zwischen den Materialien geschaffen wird, die nur durch Zerstörung des Materials, hier des Myzelverbundes oder der Gewebelage, gelöst werden kann. Diese Art der Verbindung ist besonders vorteilhaft für die Schaffung von langlebigen und stabilen Mehrlagenverbundelementen, da sie eine integrale Struktur mit homogenen Eigenschaften über die Verbindungsstelle hinaus ermöglicht.

"Formschlüssig", im Rahmen dieser Erfindung, beschreibt eine Verbindungsart, bei der die Verbindung zwischen den Komponenten durch die ineinandergreifende Form oder Gestaltung der beteiligten Teile erreicht wird. Dies bedeutet, dass die physikalische Konfiguration eines Elements in eine entsprechende Negativform eines anderen Elements passt, wodurch eine Verbindung durch mechanische Verzahnung ohne direkten Materialkontakt entsteht. Formschlüssige Verbindungen sind durch ihre hohe mechanische Festigkeit und die Fähigkeit, Scherkräfte aufzunehmen, charakterisiert. In der vorliegenden Erfindung ermöglicht die formschlüssige Verbindung zwischen den Abstandselementen und dem Myzelverbund bzw. den Gewebelagen eine effektive Lastübertragung und Stabilität des Mehrlagenverbundelements.

In einer bevorzugten Ausführungsform weist das Mehrlagenverbundelement im Sinne der vorliegenden Erfindung eine Dichte im Bereich von 50 bis 500 kg/m³, bevorzugter zwischen 100 bis 250 kg/m³ auf. Die Dichte wird bevorzugt durch die Auswahl des Substratmaterials, Füllpartikel, das Volumen und die Art der inokulierten Pilzsporen sowie durch die spezifischen Bedingungen während des Wachstums- und Inkubationsprozesses bestimmt. Insbesondere bevorzugt liegt die Dichte in dem numerischen Bereich, der sich aus der Kombination von zwei beliebigen der folgenden Endpunktwerte ergibt: 50 kg/m³, 70 kg/m³, 80 kg/m³, 90 kg/m³, 100 kg/m³, 110 kg/m³, 120 kg/m³, 130 kg/m³, 140 kg/m³, 150 kg/m³, 200 kg/m³, 250 kg/m³, 300 kg/m³, 350 kg/m³, 400 kg/m³, 450 kg/m³ und 500 kg/m³. Diese Dichtenbereiche erlaubt eine präzise Anpassung der Eigenschaften des Mehrlagenverbundelements an spezifische Anforderungen, indem die Dichte gezielt eingestellt wird, um optimale Ergebnisse hinsichtlich Belastbarkeit, Wärmeisolierung und Gewicht zu erzielen. Die Dichte wird bevorzugt aus Volumen und Gewicht des Mehrlagenverbundelementes bestimmt, bevorzugt mittels eines Pyknometers.

In einigen alternativen Ausführungsformen können Mehrlagenverbundelemente eine Dichte im Bereich von 100 bis 175 kg/m³ aufweisen, Mehrlagenverbundelemente in diesem Bereich zeichnen sich insbesondere durch eine geringe Wärmeleitfähigkeit aus.

In weiteren alternativ bevorzugten Ausführungsformen können Mehrlagenverbundelemente eine Dichte im Bereich von 175 bis 250 kg/m³ aufweisen, Mehrlagenverbundelemente in diesem Bereich sind insbesondere für die Verwendung in konstruktiven Leichtbauelemente mit hoher Druckfestigkeit geeignet.

Bevorzugt weist das Mehrlagenverbundelement eine Wärmeleitfähigkeit im Bereich von 0.02 bis 0.15 W/(m·K), bevorzugter zwischen 0.03 bis 0.10 W/(m·K), ganz besonders im Bereich von 0.03 bis 0.05 W/(m·K) auf. Diese spezifischen Werte für die Wärmeleitfähigkeit unterstreichen die herausragende Isolationseffizienz des Mehrlagenverbundelements, die es ermöglicht, thermische Energie effektiv zu regulieren und Wärmeverluste zu minimieren. Die Wärmeleitfähigkeit wird maßgeblich durch die Beschaffenheit des Myzelverbundes, die Auswahl und Anordnung der Gewebelagen sowie durch die Dichte des Materials bestimmt. Die Wärmeleitfähigkeit wird bevorzugt mittels einer Nadelsonde oder einem Wärmeflusssensor bestimmt.

In einigen Ausführungsformen weist das Mehrlagenverbundelement eine Druckfestigkeit, insbesondere entlang der Z-Achse (siehe Fig. 2), im Bereich von 0,17 bis 8,0 N/mm², bevorzugter 2,0 bis 6,0 N/mm², auf. Insbesondere bevorzugt liegt die Dichte in dem numerischen Bereich, der sich aus der Kombination von zwei beliebigen der folgenden Endpunktwerte ergibt: 0,17 N/mm², 0,17 N/mm², 0,25 N/mm², 0,5 N/mm², 0,75 N/mm², 1 N/mm², 1,5 N/mm², 2 N/mm², 2,5 N/mm², 3 N/mm², 3,5 N/mm², 4 N/mm², 4,5 N/mm², 5 N/mm², 5,5 N/mm², 6 N/mm², 6,5 N/mm², 7 N/mm², 7,5 N/mm² und 8 N/mm². Diese Spanne der Druckfestigkeit verdeutlicht die hohe Belastbarkeit des Mehrlagenverbundelements unter kompressiven Kräften, was es besonders geeignet für tragende und strukturelle Anwendungen macht, bei denen eine hohe Resistenz gegenüber Druckbelastungen erforderlich ist. Die Druckfestigkeit wird typischerweise durch einen Druckfestigkeitstest bestimmt, bei dem eine Probe des Materials in einer speziellen Prüfmaschine einem kontinuierlich steigenden Druck ausgesetzt wird, bis die Probe versagt oder sich bis zu einem bestimmten Grad verformt, im Kontext dieser Erfindung bis 50 % der ursprünglichen Breite der Probe der Mehrlagenverbundelements. Die maximale Druckkraft, die die Probe aushält, geteilt durch die Querschnittsfläche der Probe, ergibt die Druckfestigkeit. Geeignete Prüfmaschinen sind dem Fachmann bekannt und Beispielsweise in der DIN EN 12390-4 beschrieben.

Bevorzugt weist das Mehrlagenverbundelement eine Zugfestigkeit, insbesondere entlang der X und/oder Y- Achse, im Bereich von 250 bis 4000 N/mm², bevorzugter zwischen 300 und 3800 N/mm² auf. In einigen bevorzugten Ausführungsformen der Erfindungen die Zugfestigkeit in dem numerischen Bereich liegt, der sich aus der Kombination von zwei beliebigen der folgenden Endpunktwerte ergibt: 250 N/mm², 275 N/mm², 300 N/mm², 325 N/mm², 350 N/mm², 375 N/mm², 400 N/mm², 425 N/mm², 450 N/mm², 475 N/mm², 500 N/mm², 600 N/mm², 700 N/mm², 800 N/mm², 900 N/mm², 1.000 N/mm², 1.250 N/mm², 1.500 N/mm², 1.750 N/mm², 2.000 N/mm², 2.250 N/mm², 2.500 N/mm², 2.750 N/mm², 3.000 N/mm², 3.250 N/mm², 3.500 N/mm², 3.750 N/mm² und 4.000 N/mm². Die Zugfestigkeit des Mehrlagenverbundelements wird insbesondere bevorzugt durch die Zugfestigkeit der Gewebelagen des Mehrlagengewebes begrenzt. Besonders bevorzugte Beispiele für die Zugfestigkeit der Fasern, Fäden, umsponnenen Fäden oder Garne der Gewebelage sind Polyester- (450 bis 750 N/mm²), Aramid- (2.400 - 3.000 N/mm²), Basaltfaser- (bis zu 3.750 N/mm²), Baumwoll- (330 bis 585 N/mm²), Flachs- (345 bis 585 N/mm²), Hanffaser-(690 bis 1.000 N/mm²), Jutefaser- (N/mm²), Seiden- (650 bis 750 N/mm²), Glas- (E-Glas-) (1.800 N/mm²), Kohlefasergewebe (2.400 bis 3.400 N/mm²).

### Mehrlagengewebe

Mehrlagengewebe im Sinne der vorliegenden Erfindung, auch als Mehrwandgewebe bezeichnet, umfassen zumindest zwei, bevorzugt 2 bis 10, voneinander beabstandete, mit Abstandselementen verbundene Gewebelagen, Gewebelagen, auch als Wandschichten bezeichnet, die dazu eingerichtet sind einen Zwischenraum zwischen den verbundenen Gewebelagen aufzuspannen. Besonders bevorzugt ist jedoch ein Zweiwandgewebe, umfassend zwei Gewebelagen. In einigen bevorzugten Ausführungsformen kann das Mehrlagengewebe eine Anzahl an Gewebelagen aufweisen, die in dem numerischen Bereich liegt, der sich aus der Kombination von zwei beliebigen der folgenden Endpunktwerte ergibt: 2, 3, 4, 5, 6, 7, 8, 9, 10. Dies ermöglicht eine anwendungsspezifische Anpassung der Anzahl der Gewebelagen, um den jeweiligen Anforderungen gerecht zu werden. Insbesondere für Mehrlagenverbundelemente, die in strukturellen Anwendungen oder unter hohen Belastungen eingesetzt werden, kann eine erhöhte Anzahl von Gewebelagen zu einer signifikanten Steigerung der Stabilität beitragen. Dieser modulare Ansatz bietet den technischen Vorteil einer flexiblen Anpassung der Wandstärken des Mehrlagenverbundelements, um optimale Leistungseigenschaften unter verschiedenen Einsatzbedingungen zu gewährleisten. Besonders bevorzugt sind Mehrlagengewebe mit zwei Gewebelagen, da in diesem Fall beide Gewebelagen jeweils eine äußere Lage des Mehrlagenverbundelementes darstellen, was insbesondere das Inkubieren des Myzelverbundes im Sinne des vorliegenden Verfahrens erleichtert.

Erfindungsgemäß liegen die Abstandselemente im Zustand der Wirkverbundenheit mit dem Myzelverbund gestreckt oder unter Spannung gesetzt vor, wodurch ein definierter Zwischenraum zwischen den verbundenen Gewebelagen aufgespannt wird. Dies erfolgt insbesondere durch Ausführung des erfindungsgemäßen Verfahrens, bei dem es zu einer Ausdehnung des Myzelmaterials kommt. Dabei muss beachtet werden, dass die Aufarbeitung nach der Inkubation, insbesondere die Trocknung so erfolgt, dass es zu keiner übermäßigen Schrumpfung des Myzelverbundes kommt.

In einer bevorzugten Ausführungsform des Mehrlagenverbundelements weisen die beabstandeten Gewebelagen eine Beabstandung im Bereich von 0,1 cm bis 100 cm, bevorzugter zwischen 0,5 cm und 50 cm, am bevorzugtesten 0,5 bis 15 cm, auf, die der durchschnittlichen Länge der gestreckten Abstandselemente entspricht. Es versteht sich, dass die Höhe (Z) des Zwischenraums in diesem Fall ebenfalls der Höhe der gestreckten Abstandselemente und damit der Beabstandung entspricht. Insbesondere bevorzugt kann die Beabstandung in dem numerischen Bereich liegen, der sich aus der Kombination von zwei beliebigen der folgenden Endpunktwerte ergibt: 0.1 cm, 0.25 cm, 0.5 cm, 0.75 cm, 1 cm, 2 cm, 3 cm, 4 cm, 5 cm, 6 cm, 7 cm, 8 cm, 9 cm, 10 cm, 11 cm, 12 cm, 13 cm, 14 cm, 15 cm, 16 cm, 17 cm, 18 cm, 19 cm, 20 cm, 25 cm, 30 cm, 35 cm, 40 cm, 45 cm, 50 cm, 55 cm, 60 cm, 65 cm, 70 cm, 75 cm, 80 cm, 85 cm, 90 cm, 95 cm, 100 cm.

Es erweist sich als besonders vorteilhaft, die Beabstandung bzw. die Höhe (Z) des Zwischenraums im Bereich von 0,5 bis 15 cm zu halten. In diesem Intervall ist die natürliche Sauerstoffversorgung des Myzels bzw. des Pilzes während der Inkubationsphase ohne den Einsatz von technischen Hilfsmitteln sichergestellt. Mehrlagenverbundelemente, die eine Beabstandung zwischen den Gewebelagen von 15 cm bis zu 100 cm aufweisen, sind ebenfalls realisierbar. Für diese Konfigurationen kann eine externe Sauerstoffzufuhr erforderlich sein, um eine optimale Inkubation zu gewährleisten. Ein solches Verfahren könnte analog zu dem in der Patentschrift WO 2019/226823 A1 beschriebenen Ansatz implementiert werden, wodurch eine effiziente Sauerstoffversorgung auch bei größeren Zwischenräumen ermöglicht wird. Dies ermöglicht die Nutzung erweiterter Designoptionen und Anwendungsgebiete für Mehrlagenverbundelemente, indem es die Flexibilität in Bezug auf die Dimensionierung des Zwischenraums erhöht, ohne die Lebensfähigkeit oder das Wachstum des Myzels zu beeinträchtigen.

Zwei "beabstandete" Gewebelagen können flächig, planar, windschief-planar bei bestimmungsgemäßem Gebrauch angeordnet sein.

In einigen bevorzugten Ausführungsformen des Mehrlagenverbundelements im Sinne der vorliegenden Erfindung entspricht die Bruchgrenze des Mehrlagengewebes derjenigen des Mehrlagenverbundelements. Dem Fachmann ist bekannt, dass unter einer "Bruchgrenze" der Punkt in einem Spannungs-Dehnungs-Diagramm, wie es durch einen Zugtest nach DIN 50125 ermittelt werden kann. Bevorzugt wird diese an einem rechteckigen Probekörper (Form E) des Mehrlagenverbundelementes bestimmt. Dies gewährleistet eine hohe Stabilität bei Anwendungen, bei denen hohe Zugbelastungen auftreten, z.B. Fassaden- oder Konstruktionselemente, bei denen Zugbelastungen durch Winddruck oder Wärmedehnung auftreten, Dachkonstruktionen unter Schneelast, Rahmenkonstruktionen im Wohnmobil- und Caravanbau. Die Verbindung aus einem Myzelverbund

Ferner können die erfindungsgemäßen Leichtbauelemente an den außenliegenden Gewebeflächen vernäht, zu verklebt oder verschweißt werden.

In besonders bevorzugten Ausführungsformen ist das Mehrlagengewebe luft-, licht- und feuchtigkeitsdurchlässig. Dies erlaubt das effiziente Inkubieren des Myzelverbundes in einem Zwischenraum des Mehrlagengewebes.

Selbstverständlich muss zu in diesen Ausführungsformen zumindest eine vom Mehrlagengewebe umfasste Gewebelage luft-, licht- und feuchtigkeitsdurchlässig sein. "Luft-, licht- und feuchtigkeitsdurchlässig" im Sinne der Erfindung bedeutet, dass ein gewisser Anteil, vorzugsweise mindestens 5 %, der Luft, des Lichts und der Feuchtigkeit aus der Umgebung des mehrlagigen Gewebes durch mindestens eine Gewebelage in den Zwischenraum hindurchtreten kann.

Vorteilhaft kann in einigen Ausführungsformen zumindest eine Gewebelage eine Beschichtung und/oder Versiegelung aufweisen. Diese dient zur Erhöhung der Resistenz gegenüber Luftfeuchtigkeit und kann das Wasserzeihen des Myzelverbundes unterdrücken.

Weiterhin kann die Beschichtung und/oder Versiegelung bevorzugt antimikrobielle und/oder antifugale und/oder UV-absorbierende Substanzen umfassen, die die Lebensdauer der Mehrlagenverbundelemente zu erhöhen.

In einer bevorzugten Ausführungsform sind die Gewebelagen des Mehrlagengewebes in dieselbe Richtung ausgerichtet, wobei sich die Ausrichtung auf die Ausrichtung der von den Gewebelagen umfassten oder darin angeordneten Fasern, Fäden oder Garne bezieht. In anderen bevorzugten Ausführungsformen stehen die Gewebelagen in einem gewinkelt zueinander, vorzugsweise 45°, 90°, 135° oder 180°. Dadurch kann insbesondere die Zugbelastung des Mehrlagengewebes and die gegebene konstruktiven Anforderungen angepasst werden. Im Kontext dieser Erfindung werden die angegebene Werte für Materialeigenschaften, wie Zugfestigkeit, der Mehrlagenverbundelemente bevorzugt für Mehrlagengewebe mit gleichausgerichtete Gewebelagen, die in X oder Y - Richtung ausgerichtet sind, bestimmt.

### Gewebelagen und Abstandselemente

Im Kontext der vorliegenden Erfindung bezieht sich der Begriff "Gewebelage" auf eine einzelne Schicht oder Ebene innerhalb eines Mehrlagenverbundelements, die aus einem systematisch verflochtenen Material besteht, um eine flächige Struktur zu bilden. Gewebe werden durch das Verflechten von Fäden oder Garnen in einem Muster hergestellt, wobei die zwei grundlegenden Komponenten die Kette (Längsfäden) und der Schuss (Querfäden) sind, dem Fachmann sind verschiedene Ausführungsformen von Geweben bekannt. Im Kontext dieser Erfindung dient eine Gewebelage unter anderem als strukturelle Komponente, die zur mechanischen Festigkeit, Isolation sowie der räumlichen Abgrenzung des Verbundelements beiträgt. In einem Mehrlagenverbundelement können mehrere Gewebelagen übereinander angeordnet sein, um die gewünschten Eigenschaften zu erzielen. Jede Lage ist darauf ausgelegt, in Synergie mit anderen Gewebelagen und Abstandselementen und dem Myzelverbund zur Gesamtperformance und Funktionalität des Verbundes beizutragen.

In besonders bevorzugten Ausführungsformen des Mehrlagenverbundelements umfassen oder bestehen die Gewebelagen und/oder die Abstandselemente unabhängig voneinander aus einem Material ausgewählt aus der Liste umfassend Natur-, Mineral-, Hybrid-, Synthetik- und Metallfasern. Durch die Auswahl spezifischer Materialien lässt sich eine optimale Balance zwischen der Kompatibilität mit Myzelwachstum und der Gewährleistung struktureller Integrität erreichen. Die Materialauswahl ermöglicht die Anpassung der physikalischen, mechanischen oder ästhetischen Eigenschaften des Mehrlagenverbundelements an spezifische Anforderungen. Das Material wird in einer entsprechend geeigneten Form bereitgestellt, bei Abstandselementen vorzugsweise in Form von Drähten, Fäden, Garnen, beschichteten Fäden oder Fasern, bei Gewebelagen vorzugsweise als Gewebe und/oder Gewirke, das die entsprechenden Materialien vorzugsweise in Form von Drähten, Fäden, Garnen, beschichteten Fäden oder Fasern umfasst.

Unter "Naturfasern" werden im Kontext dieser Erfindung insbesondere Fasern verstanden, die aus pflanzlichen oder tierischen Quellen gewonnen werden. Bevorzugte Beispiele umfassen Baumwolle, Flachs, Hanf, Seide, Jute, Sisal Kokosfaser oder Wolle. Der technische Effekt der Verwendung von Naturfasern in Gewebelagen und/oder Abstandselementen liegt in ihrer Umweltverträglichkeit, Biokompatibilität und ihrer Fähigkeit, Luftdurchlässigkeit und Feuchtigkeit zu regulieren.

Im Zusammenhang mit der vorliegenden Erfindung sind Mineralfasern Fasern, die aus anorganischen Materialien wie Glas, Basalt, Schlacke oder Keramik hergestellt werden. Bevorzugte Beispiele sind Glasfasern, Basaltfasern und Keramikfasern. Der technische Effekt der Verwendung von Mineralfasern liegt in ihrer hohen mechanischen Festigkeit, thermischen Beständigkeit und Schalldämmung. Keramikfasern bieten zusätzlich eine exzellente Hitzebeständigkeit und chemische Stabilität, was die strukturelle und funktionelle Performance von Verbundelementen verbessert. Besonders bevorzugt sind in dem Kontext der Gewebelagen Basaltfasern, die eine hohe mechanische Zugfestigkeit, thermische Beständigkeit, chemische Stabilität und Umweltverträglichkeit aufweisen, da das Herstellungsverfahren aus natürlichem Basaltgestein in der Regel energieeffizienter ist als beispielsweise die Herstellung von Glasfasern.

Unter Synthese- oder Synthetikfasern werden künstlich hergestellte Fasern aus Polymeren oder Pyrolyseprodukte aus Polymeren, die chemisch synthetisiert werden, verstanden. Bevorzugte Beispiele umfassen Polyamid, insbesondere Nylon und Aramide, Polyester, Polyacryl, Polyacrylnitril und Kohlefasern. Der technische Effekt der Verwendung von Synthesefasern liegt in ihrer hohen Zugfestigkeit, Langlebigkeit und Beständigkeit gegenüber Chemikalien und Feuchtigkeit, was die Lebensdauer und Witterungsbeständigkeit des Mehrlagenverbundelementes erhöhen kann.

Metallfasern oder Metallfäden sind Fasern oder Fäden, die aus Metall oder metallischen Legierungen bestehen. Der technische Effekt der Verwendung von Metallfasern in Gewebelagen und/oder Abstandselementen liegt in ihrer außergewöhnlichen Festigkeit, elektrischen Leitfähigkeit und Hitzebeständigkeit. Diese Eigenschaften tragen zur Verbesserung der strukturellen Stabilität und Funktionalität des Mehrlagenverbundelements bei, insbesondere in Anwendungen, die eine hohe Belastbarkeit oder spezifische funktionelle Eigenschaften wie Feuerresistenz oder elektrische Leitfähigkeit oder Isolierung gegen elektrische Felder erfordern.

Hierin beziehen sich Hybridfasern auf Materialien, die aus einer Kombination von zwei oder mehreren Fasertypen zusammengesetzt sind, um die vorteilhaften Eigenschaften jedes Materials zu nutzen. Sie können alternativ bevorzugt auch Beschichtungen oder Kaschierungen aufweisen. Der technische Effekt von Hybridfasern liegt in der maßgeschneiderten Einstellung von Eigenschaften wie Festigkeit, Flexibilität und Haltbarkeit, die durch die Kombination verschiedener Fasertypen erreicht wird.

Erfindungsgemäß sind die Gewebelagen durch Abstandselemente, bevorzugt ausgewählt aus der Liste umfassend textile Verbindungsfäden und/oder -garne, und/oder metallische Verbindungsdrähte, miteinander verbunden. Diese verbindenden Fäden können mit den beiden Geweben verwoben, verstrickt oder verschmolzen sein. Ganz besonders bevorzugt werden die Abstandselemente als textile Abstandselemente, insbesondere als Fäden oder Garne ausgestaltet. Dies erlaubt das Verweben oder Verstricken der Abstandselemente mit den Gewebelagen, was für eine stabile Verbindung zwischen Gewebelagen und Abstandselementen führt.

In einigen besonders bevorzugten Ausführungsformen können sowohl die Gewebelage und/oder die Abstandselemente aus einem biokompatiblen Material, bevorzugt als Fasern, Garne, Fäden ausgestaltet, insbesondere Naturfasern, bevorzugt ausgewählt aus der Liste umfassend Baumwolle, Flachs, Hanf, Jute, Sisal Kokosfaser oder Wolle, oder geeigneten biobasierten Polymeren, bevorzugt ausgewählt aus Polylactiden oder Polyhydroxyalkanoaten, bestehen. Bestimmte Naturfasern und biobasierten Polymere können von Myzel durchwachsen werden und dabei ihre strukturelle Integrität zu einem gewissen Grad beibehalten. Diese Fasern bieten eine sowohl eine Matrix als auch potenzielle Nährstoffquelle, die das Myzelwachstum unterstützt.

In besonders bevorzugten Ausführungsformen können Hybridmaterialien, die eine Kombination aus Synthetikfasern, Naturfasern, modifizierten Naturfasern und metallischen Drähten umfassen, als Materialien sowohl für die Gewebelagen als auch für die Abstandselemente eingesetzt werden. Diese Zusammensetzung ermöglicht eine optimale Balance zwischen der Kompatibilität mit Myzelwachstum und der Gewährleistung struktureller Integrität.

In einigen bevorzugten Ausführungsform sind die Abstandselemente nicht nur als einzelne Elemente, sondern beispielsweise gebündelt oder in Form von abstandshaltenden Stegen, ausgebildet.

In einigen bevorzugten Ausführungsformen der Erfindung weist das Mehrlagengewebe eine Anzahl von 1.000 bis 400.000, bevorzugt 20.000 bis 240.000, Abstandselementen pro m² für jedes Paar von gegenüberliegenden Gewebelagen auf. Diese spezifische Dichte an Abstandselementen ermöglicht eine fein abgestimmte Kontrolle über die mechanischen Eigenschaften des Mehrlagenverbundelements, insbesondere in Bezug auf Flexibilität, Dichte und Zugfestigkeit. Durch die Anpassung der Anzahl der Abstandselemente kann das Verbundelement für eine Vielzahl von Anwendungen maßgeschneidert werden, von hochfesten Strukturmaterialien bis hin zu flexiblen Isolationsmaterialien.

Besonders bevorzugt sind die Abstandselemente in einem Winkel zwischen 30° und 90° angeordnet.

In einigen bevorzugten Ausführungsformen können die Abstandselemente gleichmäßig verteilt oder ungleichmäßig verteilt, zum Beispiel gebündelt vorliegen. Eine ungleichmäßige Verteilung, insbesondere in Bündeln oder in Reihen mit unterschiedlichen Abständen oder mit abgestufter Verteilung, kann in Anwendungsbereichen mit ungleichmäßiger Beanspruchung der Bauteile vorteilhaft sein, um bestimmte Bereiche, z. B. Verbindungsstellen mit anderen Bauteilen, besonders zu verstärken. Beispiele sind Aufhängungen in Außenwänden oder Dachplatten.

Polymerfasern können bevorzugt flammwidrig ausgestaltet sein. Die flammwidrige Ausrüstung kann erfolgen durch Zusatz eines Flammschutzmittels zu der Masse, aus der die Fasern erzeugt werden.

Besonders geeignet sind Gewebelagen mit einem Gewicht von 400 bis 1200 g/m². Dies stellt einen Kompromiss zwischen Stabilität des resultierenden Mehrlagenverbundelements und der Durchwachsbarkeit der Gewebelagen durch den Myzelverbund dar.

### Myzelverbund

Im Sinne der vorliegenden Erfindung wird unter einem "Myzelverbund" eine Zusammensetzung verstanden, die ein Substrat und ein das Substrat durchdringendes Myzel umfasst. Das Myzel dient als ein natürliches Bindemittel, das die Fähigkeit besitzt, das Substrat zu durchwachsen und eine bevorzugt möglichst einheitliche Matrix zu bilden. Diese Matrix ist charakterisiert durch ihre Fähigkeit, mechanische Festigkeit sowie verbesserte isolierende Eigenschaften zu den Verbundelementen beizutragen. Der Myzelverbund ist im Zwischenraum eines Mehrlagengewebes angeordnet, wobei er stoff- und formschlüssige Verbindungen mit den Gewebelagen und Abstandselementen eingeht. Das Substrat kann dabei durch das Wachstum des Myzels teilweise oder vollständig abgebaut werden.

Das "Myzel", auch als "Pilzmyzel" im Sinne der vorliegenden Erfindung verstanden, bezeichnet das Netzwerk aus feinen, fadenförmigen Strukturen, die von Pilzen gebildet werden. Diese Strukturen fungieren als die primären Wachstums- und Ernährungsorgane des Pilzes, indem sie Nährstoffe einem Substrat entnehmen und dieses dabei durchdringen. Im Kontext des Myzelverbundes dient das Myzel nicht nur als Mittel zur Nährstoffaufnahme, sondern auch als ein natürliches Klebe- und Verstärkungsmittel, das die einzelnen Bestandteile des Substrats miteinander verbindet und so zur Bildung einer festen, kohärenten Struktur beiträgt. Diese Struktur verleiht dem Mehrlagenverbundelement seine charakteristischen mechanischen und isolierenden Eigenschaften, indem sie eine homogene und zugleich robuste Matrix bildet, die effektiv Lasten tragen und thermische Energie regulieren kann.

Als geeignete Pilzsporen zum Inokulieren des Substrates werden insbesondere Pilzsporen ausgewählt aus der Liste umfassend Pleurotus ostreatus (Austernpilz), Ganoderma lucidum (Reishi oder Lackporling), Trametes versicolor (Schmetterlingstramete), Fomes fomentarius (Zunderschwamm) und Phanerochaete chrysosporium.

Zum Zwecke der Befüllung der Mehrlagengewebe können jegliche Substrate verwendet werden, die aus einem vom Pilzmyzel zersetzbaren biologischen oder synthetischen Material bestehen. Bevorzugt wird das Substrat ausgewählt aus der Liste umfassend Lignozellulose-basierte Materialien, insbesondere landwirtschaftliche Nebenprodukte und Abfälle, die reich an organischen Substanzen sind.

In einigen bevorzugten Ausführungsformen der Erfindung besteht das Substrat für den Myzelverbund aus Lignocellulose-haltigen Neben- und Abfallprodukten, wobei die Neben- und Abfallprodukte ein Material umfassen oder daraus bestehen das besonders bevorzugt ausgewählt aus der Liste umfassend oder bestehend aus, bevorzugt bestehend aus Stroh, Maisstroh, Spreustroh, Getreidestroh, Holzspäne, Hanffaser, Baumwolle, Holzsägemehl, Floh-, Chia-, Linumsamen, Reishülsen, Weizenrückstände , Hirsekorn, Weizenkleie, Kaffeesatz, Baumwollabfälle, Reishülsen, Zuckerrohrbagasse, Holzspänen, Kokosfaser. Diese beispielhaften, nicht abschließenden Materialien, die eine Auswahl der in Fachkreisen bekannten Lignozellulose-haltigen Neben- und Abfallprodukte darstellen, bieten nicht nur eine optimale Nährstoffquelle für das Wachstum des Myzels, sondern unterstützen auch die Bildung eines stabilen und dauerhaften Myzelverbundes. Durch die Auswahl spezifischer Substrate kann die Zusammensetzung und können damit die Eigenschaften des Myzelverbundes gezielt beeinflusst und an die jeweiligen Anforderungen der Mehrlagenverbundelemente angepasst werden, was eine maßgeschneiderte Optimierung von Festigkeit, Dichte, Isolationseigenschaften und biologischer Abbaubarkeit ermöglicht. Bevorzugt können Nährstofflösungen (Nährlösungen), wie zum Beispiel Kartoffel-Glucose-Bouillon (KGB) oder vergleichbare Lösungen zugegeben werden, um das gleichmäßige Wachstum des Myzels zu erlauben.

Unter "Lignocellulose-haltigen Neben- und Abfallprodukten" wird im Rahmen dieser Erfindung die Herkunft der Rohmaterialien aus beliebigen Teilen von Lignocellulose-haltigen Pflanzen verstanden. Dies umfasst alle pflanzlichen Komponenten einschließlich, aber nicht beschränkt auf Blätter, Stamm, Rinde, Stängel, Wurzeln, Früchte und Samen, der eine geeignete chemische und/oder physikalische Beschaffenheit für die beabsichtigte Anwendung besitzt. Das pflanzliche Material und/oder Stoffe und/oder ganze Pflanzen und/oder Teile von diesen können durch physikalische und/oder chemische Verfahren vorbereitet werden. Dazu gehören Prozesse wie Trocknen, Mahlen, Rösten, Extrahieren oder jede Kombination davon. Die spezifischen Verfahrensschritte sowie die physikalischen und chemischen Eigenschaften der resultierenden Lignocellulose-haltigen Neben- und Abfallprodukten sind dem Fachmann bekannt.

In einigen besonders bevorzugten Ausführungsformen wird eine Mischung aus den oben genannten Materialien eingesetzt. Selbstverständlich ist dem Fachmann bekannt, wie diese Bereitgestellt werden müssen (gehäckselt, zerkleinert, gemahlen o.Ä.), um ein effizientes Inkubieren des Myzels zu gewährleisten.

In ganz besonders bevorzugten Ausführungsformen werden regional verfügbare Materialien aus der oben genannten Liste ausgewählt, die sich den jeweiligen landwirtschaftlichen Neben- und Abfallprodukten anpassen. Dieser Ansatz ermöglicht es, die Materialauswahl auf die lokal verfügbaren Ressourcen abzustimmen und somit die Transportkosten und den damit verbundenen CO₂-Fußabdruck erheblich zu reduzieren. Durch die Nutzung von regionalen Abfallprodukten wird nicht nur eine nachhaltigere Produktion des Mehrlagenverbundelements gefördert, sondern auch die lokale Wirtschaft gestärkt und die Effizienz der Materialkreisläufe innerhalb der Region maximiert. Diese Strategie trägt zur Reduzierung von Abfall und zur Förderung einer zirkulären Wirtschaft bei, indem sie lokale Ressourcen effektiv nutzt und in wertvolle neue Produkte umwandelt.

Weiterhin können im Zwischenraum, der von den Gewebelagen aufgespannt wird, bevorzugt zusätzlich zum Myzelverbund zusätzliche funktionale Materialien bereitgestellt werden, wobei die funktionalen Materialien ausgewählt sind aus der Gruppe bestehend aus natürlichen und/oder synthetischen Bindungs-, Feuerretardations- und Wasserabweisungs-Additiven. Diese zusätzlichen Materialien ermöglichen eine maßgeschneiderte Anpassung der Verbundelemente an spezielle Anforderungen und Einsatzbedingungen, indem sie beispielsweise die strukturelle Kohäsion erhöhen, den Brandschutz verbessern oder den Schutz gegen Feuchtigkeit verstärken.

In einigen bevorzugten Ausführungsformen des Mehrlagenverbundelements können im Zwischenraum, insbesondere als Teil des Myzelverbundes, bevorzugt eingebracht über Beimischung zum inokulierten und vorinkubierten Substrat im erfindungsgemäßen Verfahren, Füllpartikel angeordnet sind, die die mechanischen Eigenschaften des Mehrlagenverbundelements beeinflussen. "Füllpartikel" beziehen sich auf feste, fein verteilte Materialien, die dazu bestimmt sind, die Eigenschaften des Myzelverbundes zu modifizieren. Die Füllpartikel bestehen aus bevorzugt pflanzlichen oder anorganischen Material, dessen Partikelgröße, Form und Oberflächenbeschaffenheit spezifiziert sind. Die mittlere Partikelgröße liegt im Bereich von 0,05 mm bis 20 mm, beispielsweise 0,1 mm, 0,2 mm, 0,3 mm, 0,4 mm, 0,5 mm, 0,6 mm, 0,7 mm, 0,8 mm, 0,9 mm, 1,0 mm, 2,0 mm, 3,0 mm, 4,0 mm, 5,0 mm, 6,0 mm, 7,0 mm, 8,0 mm, 9,0 mm, 10 mm, 11 mm, 12 mm, 13 mm, 14 mm, 15 mm, 16 mm, 17 mm, 18 mm, 19 mm bis zu 20 mm. Die Form der Partikel kann sphärisch, unregelmäßig, faserig oder jede andere geeignete Form sein. Die Partikel können optional beschichtet oder mit Zusatzstoffen versehen sein, um spezifische Eigenschaften wie Haftung, Stabilität oder Reaktivität zu verbessern. Im Kontext dieser Erfindung können Füllpartikel zur verbesserten Adhäsion des Myzelverbundes, Beeinflussung der Dichte und der Wärmeleitfähigkeitseigenschaften eingesetzt werde.

In der vorliegenden Erfindung bezieht sich "mittlere Partikelgröße" auf den durchschnittlichen Durchmesser der Füllpartikel, welche beispielsweise mit einer Gauss-(Normal-) oder Poisson-Verteilung beschrieben werden kann. Bei der Gauss-Verteilung konzentrieren sich die meisten Partikelgrößen um den Mittelwert, während bei der Poisson-Verteilung die Verteilung der Partikelgrößen durch die Poisson-Gleichung definiert wird. Die Bestimmung der mittleren Partikelgröße kann mittels Siebverfahren erfolgen, bei dem die Partikel durch ein Set von Sieben mit unterschiedlichen Maschengrößen geleitet werden, um die Größenverteilung zu ermitteln.

### Verfahren

Weiterhin betrifft die vorliegende Erfindung ein Verfahren zur Herstellung eines Mehrlagenverbundelements (1.0), umfassend zumindest die Schritte
a) Einfüllen eines inokulierten und vorinkubierten Substrats, bevorzugt eines Lignozellulose-basierten Substrats, in den Zwischenraum (2.3) eines Mehrlagengewebes (2.0), wobei das Mehrlagengewebe (2.0) in einem Rahmen, einer Greifvorrichtung oder durch ein anderweitiges Spannmittel, insbesondere ein Vakuum oder Unterdruck; gespannt ist;
b) Abdecken der Grenzflächen, bevorzugt mit einem Abdeckmittel, des Mehrlagengewebes (2.0), um die in Schritt a) erhaltene Gewebe-Substrat-Kombination von der Umwelt abzutrennen;
c) Inkubieren der Gewebe-Substrat-Kombination aus Schritt b), bevorzugt in einer Inkubationsphase mit einer bestimmten Zeit bei einer bestimmten Temperatur und/oder bei einer bestimmten Luftfeuchtigkeit, um ein vernetztes Wachstum des Myzels um das Substrat zu erreichen;
d) Abtrennen der Gewebe-Substrat-Kombination von einem Rahmen, einer Greifvorrichtung oder einem anderweitigen Spannmittel, insbesondere einem Vakuum oder Unterdruck;
e) Trocknen der Vorstufe der Gewebe-Substrat-Kombination aus Schritt i) bei einer bestimmten Temperatur, bevorzugt zwischen 35 und 110 °C, und/oder Unterdruck, bevorzugt zwischen 0,01 und 0,9 bar.

Der Schritt des Einfüllens umfasst selbstverständlich das Bereitstellen eines geeigneten inokulierten und vorinkubierten Substrats, wie aus dem Stand der Technik hinreichend beschrieben oder kommerziell erhältlich ist. Das Einfüllen kann bevorzugt über geeignete technische Hilfsmittel wie Pumpen, Schüttvorrichtungen, Pressen, Extruder oder Abfüllmaschinen durchgeführt werden. Das Mehrlagengewebe wird dabei in einem Rahmen gespannt, die äußere Form für den Myzelverbund bilden dabei die gespannten Gewebelagen. Die erfindungsgemäßen Mehrlagengewebe (2.0) werden in einem Rahmen, einer Greifvorrichtung oder durch ein anderweitiges Spannmittel gespannt, z.B. ein Vakuum oder Unterdruck.

In einigen bevorzugten Ausführungsformen umfasst das Verfahren zur Herstellung eines Mehrlagenverbundelements (1.0) zusätzliche Schritte zur Behandlung und/oder Beschichtung zumindest einer Gewebelage des Mehrlagengewebes vor oder nach der Kultivierung des Myzelverbunds, insbesondere um die Haftung zwischen den Gewebelagen (2.2) und dem Myzelverbund zu verbessern oder das Mehrlagenverbundelement auszurüsten. Dies kann vorteilige Effekte wie die Steuerung und/oder Regulierung der Feuchtigkeits- und Gasdurchlässigkeit, oder Verbesserung der Abrasion- oder chemischen Resistenz erzielen.

Das Mehrlagenverbundelement kann je nach Einsatzgebiet in seinen Eigenschaften angepasst werden, die variablen Parameter liegen zum einen in der Veränderung des Mehrlagengewebes, beispielsweise durch die Anpassung der Gewebestruktur, der Art der Abstandselemente, der Anordnung, Anzahl und Dichte der Abstandsfäden wie hierin beschrieben. Die Dicke der Bauplatten, der Dichte des Füllstoffes oder der Art der verwendeten Materialien.

Zum anderen können die Parameter des Myzelverbundes auf Basis eines Substrats und Myzel beispielsweise durch die Art des verwendeten Substrats und/oder Füllpartikel und/oder Additive und der Art des Pilzmyzels oder der zugrundeliegenden Pilzsporen angepasst werden. Durch diese Variabilität ist ein Einsatz der Mehrlagenverbundelemente in verschiedenen Gebieten möglich.

Ganz besonders bevorzugt ist die Synthese dieser Optimierungsansätze, bei der die Eigenschaften des Mehrlagengewebes, die Parameter des Myzelverbundes sowie die Parameter des erfindungsgemäßen Herstellungsverfahrens, welche in Kombination neuartige, wirkverbundene Hybridmaterialien aus Mehrlagengeweben und Myzelverbünden ergeben, vorteilig an verschiedene Isolierungs- und (Leicht-) Bauanwendungen angepasst werden können.

### Verwendung

Nachfolgend werden einige bevorzugte Einsatzgebiete der erfindungsgemäßen Mehrlagenverbundelemente erläutert.

Im Bereich der Caravan- und Wohnmobilbranche bietet das Mehrlagenverbundelement im Sinne der vorliegenden Erfindung innovative Lösungen, indem es als Material für Leichtbaumöbel, andere konstruktive Bauteile und Außenwandplatten eingesetzt wird. Die Vorteile dieser Anwendung liegen auf der Hand: Durch die Verwendung des Mehrlagenverbundelements können z.B. Tischplatten im Caravan-Bereich deutlich leichter gestaltet werden als herkömmliche Alternativen, was zu einer signifikanten Gewichtsreduktion des Gesamtfahrzeugs führt. Zudem bieten Außenwandplatten aus dem Mehrlagenverbundelement nicht nur hohe Dämmeigenschaften bei geringem Gewicht, sondern auch eine hohe Festigkeit. Ein weiterer herausragender Aspekt ist die Kreislaufwirtschaftsfähigkeit und Kompostierbarkeit der Mehrlagenverbundelemente bzw. von Elementbestandteilen, was sie zu einer umweltfreundlichen Option für die Branche macht.

Im Hallen- und Dächerbau eröffnet das Mehrlagenverbundelement neue Perspektiven, indem es als Ersatz für herkömmliche PU-Sandwichplatten für Außenwände, Innenwände oder Dächer, als Verstärkung in Aluminiumträgern und als Bodenplatte für Zwischenebenen dient. Ein entscheidender Vorteil ist die Möglichkeit, die Spannweite von Trägern von 2,5m auf 5m zu verdoppeln, wodurch eine signifikante Einsparung bei der Unterkonstruktion möglich wird, ohne dass Dämmeigenschaften und Gewicht beeinträchtigt werden. Des Weiteren ermöglicht die Verwendung des Mehrlagenverbundelements als Verstärkung in Aluträgern eine kosteneffektive Alternative zu Carbonverstärkungen. Bei Bodenplatten wird eine Gewichtsreduktion bei gleichbleibender Festigkeit erzielt, was den Bau effizienter und ressourcenschonender macht.

Für Container, Garagen, Tinyhouses und Notunterkünfte stellt das Mehrlagenverbundelement eine nachhaltige Alternative zu herkömmlichen Baustoffen bzw. Konstruktionsmaterialien dar, indem es für Seitenwände, Boden- und Dachplatten verwendet wird. Die Vorteile umfassen eine signifikante Gewichtsreduktion, besonders relevant für den Transport von Containern, und eine Einsparung bei der Konstruktion von Tinyhouses und Notunterkünften. Diese Eigenschaften machen das Mehrlagenverbundelement zu einer idealen Wahl für Projekte, die Wert auf Nachhaltigkeit, Effizienz und Reduktion von Gewicht legen.

Im Hausbau ermöglicht das Mehrlagenverbundelement den Einsatz als Wände, Zwischenwände, Isolierebene oder Dächer und adressiert damit direkt die Anforderungen an ökologisches Bauen. Die Platten sind nicht nur nachhaltig und aus nachwachsenden Rohstoffen hergestellt, sondern bieten auch herausragende Dämmeigenschaften bei niedrigem Gewicht. Die leichte und unkomplizierte Verarbeitung des Mehrlagenverbundelements erleichtert zudem den Bauablauf und reduziert die Gesamtbauzeit. Dies macht es zu einer attraktiven Option für den modernen, umweltbewussten Bauherren.

Weiterhin betrifft die vorliegende Erfindung ein Mehrlagenverbundsystem, aufweisend zumindest ein Mehrlagenverbundelement sowie zumindest eine wasserdichte und kratzfeste Oberflächenabdeckung, zum Beispiel eine Holzdekorschicht oder Kunststoffverschalung, zur Verwendung als Leichtbaumobiliar.

Auch offenbart ist ein Mehrlagenverbundsystem, aufweisend zumindest ein Mehrlagenverbundelement im Sinne der vorliegenden Erfindung, sowie zumindest eine Verstärkung und/oder eine Beschichtung, insbesondere aus Metall wie Aluminium, Weissblech oder Stahl, oder aus Kunststoff zur Verwendung als Bauelement. Diese Mehrlagenverbundsystem sind insbesondere im Einsatz für den Hallenbau und andere konstruktive Tätigkeiten geeignet.

Nachfolgend werden bevorzugte Ausführungsbeispiele der Erfindung beschrieben.

### AUSFÜHRUNGSBEISPIELE

Es werden nachfolgend Ausführungsbeispiele dargestellt. Die gewählten Abmessungen dienen lediglich der Veranschaulichung und stellen keine Einschränkung für die tatsächlichen Abmessungen und Abstände der erfindungsgemäßen Mehrlagenverbundelemente dar. Dabei zeigt
- **Fig. 1A:**: zeigt die schematische Darstellung eines erfindungsgemäßen Mehrlagengewebes (2.0) mit zwei Gewebelagen (2.1) und Abstandselementen (2.2)
- **Fig. 1B**: zeigt die schematische Darstellung eines erfindungsgemäßen Mehrlagengewebes (2.0) mit drei Gewebelagen (2.1) und Abstandselementen (2.2)
- **Fig. 1C**: zeigt die schematische Darstellung eines erfindungsgemäßen Mehrlagengewebes (2.0) mit drei Gewebelagen (2.1) und Abstandselementen (2.2) in 75 ° Winkel zur Ebene der Gewebelagen
- **Fig. 2A**:: zeigt die schematische Darstellung eines erfindungsgemäßen Mehrlagenverbundelements (1.0) mit zwei Gewebelagen (2.1) und einem Myzelverbund (3.0) in schräger Seitenansicht
- **Fig. 2B:**: zeigt die schematische Darstellung des erfindungsgemäßen Mehrlagenverbundelements aus Fig. 2A mit sichtbaren Teilbereichen (3.1) aus der Draufsicht
- **Fig. 3:**: zeigt eine Abbildung eines erfindungsgemäßen Mehrlagenverbundelements aus mit sichtbaren Teilbereichen (3.1) des durchdringenden Myzelverbundes (3.0) in der Draufsicht.
- **Fig. 4A**: zeigt die schematische Darstellung eines erfindungsgemäßen Mehrlagengewebes (2.0) mit drei Gewebelagen (2.1) und versetzt angeordneten Abstandselementen (2.2) in 75 ° Winkel zur Ebene der Gewebelagen
- **Fig. 4B**: zeigt die schematische Darstellung eines erfindungsgemäßen Mehrlagengewebes (2.0) mit drei Gewebelagen (2.1) und überkreuzten Abstandselementen (2.2) in 75 ° Winkel zur Ebene der Gewebelagen

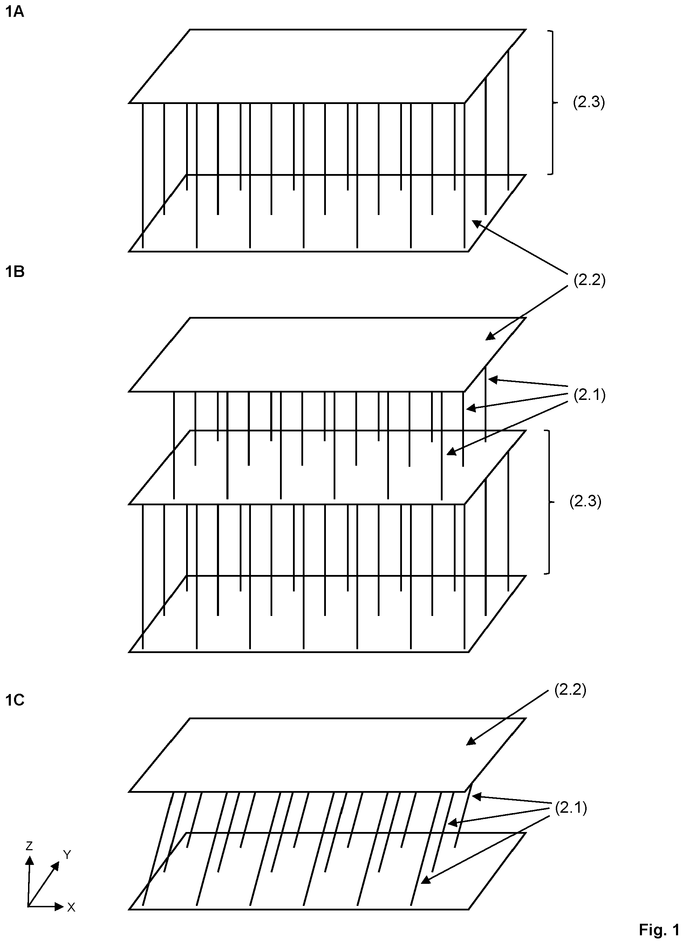

In **Fig. 1** ist eine schematische Darstellung eines erfindungsgemäßen Mehrlagengewebes (2.0) mit zwei Gewebelagen (2.1) und Abstandselementen (2.2) dargestellt. **Fig. 1A** zeigt dabei exemplarisch ein Beispiel eines zweiwandigen Mehrlagengewebes (2.0) mit zwei Gewebelagen. Die in diesem Fall vollständig gespannten Abstandselemente, die in 90° Winkel (orthogonal) zur Ebene stehen, die von der Gewebelage gespannt wird und eine gleiche Länge aufweisen. Diese definieren hierbei die Höhe des Zwischenraums (2.3), der durch die verbundenen Gewebelagen (2.2) aufgespannt wird. Die Gewebelagen (2.1) stehen in dieser idealistischen Darstellung planparallel zueinander, dem Fachmann ist jedoch bekannt, dass in realen Textilanwendungen kleine Abweichungen, z.B. zwischen 0 und 5 % der Beabstandung zustande kommen können. In **Fig. 1B** ist ein exemplarisches dreiwandiges Mehrlagengewebe (2.0) dargestellt, mit drei jeweils in Paaren und planparallel zueinander eingerichteten Gewebelagen (2.1) und gespannten Abstandselementen (2.2), die in 90° Winkel (orthogonal) zur Ebene stehen und von der Gewebelage (2.1) gespannt werden, zwischen zwei direkt gegenüberliegenden Gewebelagen (2.1), die jeweils unabhängig voneinander die gleiche Länge aufweisen. Die so aufgespannten Zwischenräume (2.3) können, wie hier schematisch dargestellt, eine unterschiedliche Höhe aufweisen. In **Fig. 1C** ist eine weitere schematische Darstellung eines erfindungsgemäßen Mehrlagengewebes (2.0) mit zwei Gewebelagen (2.1) und Abstandselementen (2.2) dargestellt, wobei die Abstandselemente hierin in einem Winkel von 75° zur Ebene ausgerichtet sind und jeweils parallel zu einem benachbarten Abstandselement (2.2) stehen.

**Fig 2** stellt eine exemplarische schematische Darstellung eines Ausschnittes des Mehrlagenverbundelements (1.0) dar. Dabei stellt **Fig. 2A** ein Mehrlagenverbundelement (1.0) mit zwei Gewebelagen (2.1) und einem Myzelverbund (3.0) in schräger Seitenansicht dar, wobei der Schnitt durch das Mehrlagenverbundelement (1.0) so gewählt ist, dass die äußerste Schicht der Abstandselemente (2.3) sichtbar ist. **Fig. 2B** zeigt die Draufsicht des erfindungsgemäßen Mehrlagenverbundelements aus **Fig. 2A** mit sichtbaren Teilbereichen (3.1) des Myzelverbundes (3.0), der die außen liegende Gewebelage (2.1) durchdrungen hat und eine formschlüssige Wirkverbindung ausbildet.

**Fig. 3** zeigt eine Abbildung eines erfindungsgemäßen Mehrlagenverbundelements aus mit sichtbaren Teilbereichen (3.1) des durchdringenden Myzelverbundes (3.0) in der Draufsicht. Hierbei stellt dieses Beispiel ein Myzelverbund mit einem Abstand des Abdeckmittels im Herstellungsverfahren von 0 mm dar, in der die Erhebungen des Webmusters der äußeren Gewebelage die Auflagefläche des Abdeckmittels darstellen und das Hervorstehen des Myzelverbundes auf 0 mm limitiert ist. Hierbei ist in Teilbereichen (3.1) eine Durchdringung des Mzyelverbundes durch die äußere Gewebelage gegeben.

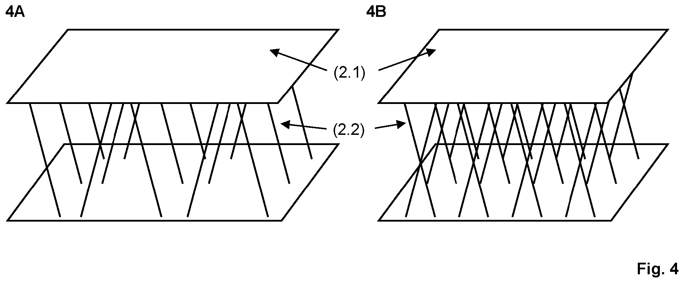

In **Fig. 4** sind zwei weitere, bevorzugte Anordnungen der Abstandselemente (2.2) dargestellt. In **Fig. 4A** ist ein erfindungsgemäßes Mehrlagengewebe (2.0) mit zwei Gewebelagen (2.1) und Abstandselementen (2.2) dargestellt, wobei die Abstandselemente (2.2) hierin in einem Winkel von 75° zur Ebene ausgerichtet sind und in einem Zickzack Anordnung (Sägezahnmuster) angeordnet sind. **Abb. 4B** zeigt eine weitere Anordnung von Abstandselementen (2.2), bei der diese in einem Winkel von 75° zur Ebene ausgerichtet und kreuzweise (überkreuzt) angeordnet sind.

### Beispiel 1: Mehrlagenverbundmaterial mit Synthesefasergewebe

Das folgende Beispiel illustriert ein exemplarisches Verfahren zur Herstellung eines erfindungsgemäßen Mehrlagenverbundmaterials auf Basis von Polyamidfasern als Gewebelagen und Aramid-Fasern als Abstandselemente. Die Beabstandung der Gewebelagen beträgt hierbei 10 cm, die Dichte der Abstandselemente 22.500 je m².
a) In einem ersten Schritt wird ein inokuliertes und vorinkubiertes Substrat in ein in diesem Fall luft-, licht- und feuchtigkeitsdurchlässiges zweiwandige Mehrlagengewebe eingefüllt, das in einem Rahmen gespannt wird. Alternativ könnte das zweiwandige Mehrlagengewebe in diesem Schritt auch durch eine Greifvorrichtung oder ein Vakuum als anderweitiges Spannmittel gespannt werden. Selbstverständlich umfasst dieser Schritt das Bereitstellen eines inokulierten und vorinkubierten Substrats auf Lignozellulose-Basis, welches kommerziell bezogen werden kann. In diesem Beispiel basiert das Substrat aus Sägespänen (2 bis 4 mm) und Stroh (gehäckselt, < 2 mm) im Verhältnis 50 Gew.-% zu 50 Gew.-%, angereichert mit Kartoffel-Glucose-Bouillon (KGB) als Nährlösung in einer Konzentration von 30 g/L, um ein nährstoffreiches Umfeld für das Pilzwachstum zu schaffen. Die Substratmischung wurde auf einen definierten Feuchtegehalt von ca. 15 % eingestellt. Das Substrat wurde unter sterilen Bedingungen mit Pilzsporen von Ganoderma lucidum inokuliert, um das Wachstum des Myzels zu initiieren. Das inokulierte Substrat wurde sorgfältig gemischt, um ein homogenes Wachstum des Myzels zu fördern. Die Mischung wurde in einer ersten Inkubationsphase für ca. 1 Woche bei Raumtemperatur (ca. 25 °C) und einer Luftfeuchtigkeit von 90 % inkubiert, um ein vernetztes Wachstum des Myzels um das Substrat herum zu erreichen.
b) Die Grenzflächen des zweiwandigen Mehrlagengewebes (2.0) werden mit einem Abdeckmittel abgedeckt, um die Gewebe-Substrat-Kombination von der Umwelt abzutrennen.
c) Die Gewebe-Substrat-Kombination aus Schritt b) wird in einer zweiten Inkubationsphase für 7 Tage bei einer Temperatur von 25 °C und einer Luftfeuchtigkeit von 90 % inkubiert, um ein weiteres vernetztes Wachstum des Myzels zu fördern.
d) Das Abdeckmittel, das die Gewebe-Substrat-Kombination von der Umwelt abgetrennt hat, wird entfernt und die Gewebe-Substrat-Kombination wird vom Rahmen bzw. der Greiffvorrichtung getrennt. Bei der alternativen Verwendung eines Vakuums wird dieses in Schritt d) abgeschaltet.
e) Die Gewebe-Substrat-Kombination aus dem vorhergehenden Schritt d) wird bei einer Temperatur von 80°C getrocknet, was zu einem myzelbasierten Lignozellulose-Verbundwerkstoff mit einem Restfeuchtigkeitsgehalt von 10 % führt.

Dieses Beispiel illustriert einen detaillierten Prozess zur Herstellung eines erfindungsgemäßen Verbundwerkstoffes als Mehrlagenverbundelement.

### Beispiel 2: Mehrlagenverbundmaterial mit Naturfasergewebe

Das folgende Beispiel illustriert ein exemplarisches Verfahren zur Herstellung eines erfindungsgemäßen Mehrlagenverbundmaterials auf Basis von Hanffasern als Gewebelagen und Baumwoll-Fasern als Abstandselemente. Die Beabstandung der Gewebelagen beträgt hierbei 5 cm, die Dichte der Abstandselemente 30.000 je m².
a) In einem ersten Schritt wird in das ein inokuliertes und vorinkubiertes Substrat (Lignozellulose-Basis, basiert auf Weizenspreu (2 bis 4 mm) und Kaffeesatz (grob, Restfeuchtigkeit etwa 15 %) im Verhältnis 70 Gew.-% zu 30 Gew.-%, angereichert mit Kartoffel-Glucose-Bouillon (KGB), definierter Feuchtegehalt von ca. 15 %, mit Pleurotus ostreatus inokuliert, 6 Tage vorinkubiert) in das hier zweiwandige Mehrlagengewebe mit Hanffasern als Gewebelagen und Baumwoll-Fasern als Abstandselemente eingefüllt, das in einem Rahmen oder durch eine Greifvorrichtung oder ein Vakuum gespannt wird.
b) Die Grenzflächen des zweiwandigen Mehrlagengewebes (2.0) werden mit einem Abdeckmittel abgedeckt, um die Gewebe-Substrat-Kombination von der Umwelt abzutrennen.
c) Die Gewebe-Substrat-Kombination aus Schritt b) wird in einer zweiten Inkubationsphase für 6 Tage bei einer Temperatur von 25 °C und einer Luftfeuchtigkeit von 90 % inkubiert, um ein weiteres vernetztes Wachstum des Myzels zu fördern.
d) Das Abdeckmittel, das die Gewebe-Substrat-Kombination von der Umwelt abgetrennt hat, wird entfernt und die Gewebe-Substrat-Kombination wird vom Rahmen bzw. der Greiffvorrichtung getrennt. Bei der alternativen Verwendung eines Vakuums wird dieses in Schritt d) abgeschaltet.
e) Die Gewebe-Substrat-Kombination aus dem vorhergehenden Schritt d) wird bei einer Temperatur von 80 °C getrocknet, was zu einem myzelbasierten Lignozellulose-Verbundwerkstoff mit einem Restfeuchtigkeitsgehalt von 10 % führt.

Dieses Beispiel illustriert einen detaillierten Prozess zur Herstellung eines erfindungsgemäßen Verbundwerkstoffes als Mehrlagenverbundelement.

### BEZUGSZEICHENLISTE

- (1.0): Mehrlagenverbundelement
- (2.0): Mehrlagengewebe
- (2.1): Gewebelagen
- (2.2): Abstandselemente
- (2.3): Zwischenraum
- (3.0): Myzelverbund
- (3.1): Teilbereiche

## Patentansprüche

1. **Mehrlagenverbundelement (1.0),** als Leichtbauelement für konstruktive und isolierende Anwendungen, umfassend zumindest
- ein Mehrlagengewebe (2.0), umfassend zumindest zwei zueinander beabstandete, mit Abstandselementen (2.1) verbundene Gewebelagen (2.2), die dazu eingerichtet sind, einen Zwischenraum (2.3) zwischen den verbundenen Gewebelagen (2.2) aufzuspannen,
- ein Myzelverbund (3.0), umfassend ein Substrat und ein das Substrat durchdringendes Myzel,
**gekennzeichnet dadurch, dass**
der Myzelverbund (3.0) im Zwischenraum (2.3), der durch die zumindest zwei Gewebelagen (2.2) aufgespannt wird, angeordnet ist, und dass
der Myzelverbund (3.0) jeweils in Teilbereichen (3.1) form- und/oder stoffschlüssig mit zumindest zwei gegenüberliegenden Gewebelagen (2.2) des Mehrlagengewebes und form- und/oder stoffschlüssig mit den Abstandselementen (2.1) verbunden ist, und
das Mehrlagengewebe (2.0) eine Anzahl von 1.000 bis 400.000 Abstandselementen (2.1) pro m² für jedes Paar von gegenüberliegenden Gewebelagen aufweist.

2. Mehrlagenverbundelement (1.0) nach Anspruch 1, wobei die Bruchgrenze, bestimmt mittels eines Zugtests nach DIN 50125, des Mehrlagengewebes derjenigen des Mehrlagenverbundelements entspricht.

3. Mehrlagenverbundelement (1.0) nach einem der Ansprüche 1 oder 2, wobei die Beabstandung der Gewebelagen (2.2) im Bereich von 0,1 cm bis 100 cm liegt.

4. Mehrlagenverbundelement (1.0) nach einem der Ansprüche 1 bis 3, wobei die Gewebelagen (2.2) und/oder die Abstandselemente (2.1) unabhängig voneinander ein Material umfassen, das aus der Liste bestehend aus Natur-, Mineral-, Hybrid-, Synthetik- und Metallfasern ausgewählt ist.

5. Mehrlagenverbundelement (1.0) nach einem der Ansprüche 1 bis 4, wobei das Substrat für den Myzelverbund (3.0) aus Lignocellulose-haltigen Neben- und Abfallprodukten besteht, wobei die Neben- und Abfallprodukte ein Material umfassen, das ausgewählt ist aus der Liste bestehend aus Stroh, Maisstroh, Spreustroh, Getreidestroh, Holzspäne, Hanffaser, Baumwolle, Holzsägemehl, Floh-, Chia-, Linumsamen, Reishülsen, Weizenrückstände , Hirsekorn, Weizenkleie, Kaffeesatz, Baumwollabfälle, Reishülsen, Zuckerrohrbagasse, Holzspänen, Kokosfaser oder eine Kombination davon.

6. Mehrlagenverbundelement (1.0) nach einem der Ansprüche 1 bis 5, wobei das Mehrlagenverbundelement (1.0) eine Dichte im Bereich von 50 bis 500 kg/m³ aufweist, bestimmbar aus dem Verhältnis von Volumen zu Gewicht des Mehrlagenverbundelementes (1.0).

7. Mehrlagenverbundelement (1.0) nach einem der Ansprüche 1 bis 6, wobei das Mehrlagenverbundelement (1.0) eine Druckfestigkeit im Bereich von 0,17 bis 8,0 N/mm² aufweist, bestimmt durch eine Prüfmaschine bei kontinuierlich steigendem Druck bis zum Versagen oder bis zu einer Verformung auf 50 % der ursprünglichen Breite der Probe der Mehrlagenverbundelements (1.0).

8. Mehrlagenverbundelement (1.0) nach einem der Ansprüche 1 bis 7, wobei das Mehrlagenverbundelement (1.0) eine Gewebelage mit einer Zugfestigkeit im Bereich von 250 bis 4.000 N/mm² aufweist, wobei die Fasern, Fäden, umsponnenen Fäden oder Garne der Gewebelage ausgewählt sind aus der Liste bestehend aus Polyester- (450 bis 750 N/mm²), Aramid- (2.400 - 3.000 N/mm²), Basaltfaser- (bis zu 3.750 N/mm²), Baumwoll- (330 bis 585 N/mm²), Flachs- (345 bis 585 N/mm²), Hanffaser-(690 bis 1.000 N/mm²), Jutefaser- (N/mm²), Seiden- (650 bis 750 N/mm²), Glas- (E-Glas-) (1.800 N/mm²), Kohlefasergewebe (2.400 bis 3.400 N/mm²).

9. Mehrlagenverbundelement (1.0) nach einem der Ansprüche 1 bis 8, wobei im Zwischenraum (2.3) zusätzlich zum Myzelverbund zusätzliche funktionale Materialien bereitgestellt werden, wobei die funktionalen Materialien ausgewählt sind aus der Gruppe bestehend aus natürlichen und/oder synthetischen Bindungs-, Feuerretardations- und Wasserabweisungs-Additiven.

10. Mehrlagenverbundelement (1.0) nach einem der Ansprüche 1 bis 9, wobei im Zwischenraum (2.3) Füllpartikel angeordnet sind.

11. Mehrlagenverbundelement (1.0) nach einem der Ansprüche 1 bis 10, wobei zumindest eine Gewebelage eine Beschichtung und/oder Versiegelung aufweist.

12. Mehrlagenverbundelement (1.0) nach Anspruch 11, wobei die Beschichtung und/oder Versiegelung antimikrobielle und/oder antifugale und/oder UV-absorbierende Substanzen umfasst.

13. Verfahren zur Herstellung eines Mehrlagenverbundelements (1.0) nach einem der Ansprüche 1 bis 12, umfassend zumindest die Schritte
a) Einfüllen eines inokulierten und vorinkubierten Substrats, bevorzugt eines Lignozellulose-basierten Substrats, in den Zwischenraum (2.3) eines Mehrlagengewebes (2.0), wobei das Mehrlagengewebe (2.0) in einem Rahmen, einer Greifvorrichtung oder durch ein anderweitiges Spannmittel gespannt ist;
b) Abdecken der Grenzflächen des Mehrlagengewebes (2.0), um die in Schritt a) erhaltene Gewebe-Substrat-Kombination von der Umwelt abzutrennen;
c) Inkubieren der Gewebe-Substrat-Kombination aus Schritt b);
d) Abtrennen der Gewebe-Substrat-Kombination von einem Rahmen, einer Greifvorrichtung oder einem anderweitigen Spannmittel;
e) Trocknen der Vorstufe der Gewebe-Substrat-Kombination aus Schritt i) bei einer bestimmten Temperatur und/oder Unterdruck.

14. Mehrlagenverbundsystem als Leichtbaumobiliar, aufweisend zumindest ein Mehrlagenverbundelement nach einem der Ansprüche 1 bis 12, sowie zumindest eine wasserdichte und kratzfeste Oberflächenabdeckung.

15. Mehrlagenverbundsystem als Bauelement, aufweisend zumindest ein Mehrlagenverbundelement nach einem der Ansprüche 1 bis 12, sowie zumindest eine Verstärkung und/oder eine Beschichtung, insbesondere aus Metall wie Aluminium, Weißblech oder Stahl oder aus Kunststoff.
